(19) **Europäisches Patentamt / European Patent Office / Office européen des brevets**

(11) **EP 1 797 195 B1**

(12) **EUROPEAN PATENT SPECIFICATION**

(45) Date of publication and mention
of the grant of the patent:
**22.12.2010 Bulletin 2010/51**

(21) Application number: **05784796.4**

(22) Date of filing: **26.09.2005**

(51) Int Cl.:
*C12Q 1/68* (2006.01)       *G01N 21/64* (2006.01)

(86) International application number:
**PCT/IB2005/053168**

(87) International publication number:
**WO 2006/038149 (13.04.2006 Gazette 2006/15)**

(54) **METHOD AND APPARATUS FOR THE DETECTION OF LABELING ELEMENTS IN A SAMPLE**

VERFAHREN UND VORRICHTUNG ZUR DETEKTION VON MARKIERUNGSELEMENTEN IN EINER PROBE

PROCEDE ET APPAREIL POUR LA DETECTION D'ELEMENTS DE MARQUAGE DANS UN ECHANTILLON

(84) Designated Contracting States:
**AT BE BG CH CY CZ DE DK EE ES FI FR GB GR HU IE IS IT LI LT LU LV MC NL PL PT RO SE SI SK TR**

(30) Priority: **01.10.2004 EP 04104834**

(43) Date of publication of application:
**20.06.2007 Bulletin 2007/25**

(73) Proprietor: **Koninklijke Philips Electronics N.V.**
**5621 BA Eindhoven (NL)**

(72) Inventors:
• **WIMBERGER-FRIEDL, Reinhold**
**52066 Aachen (DE)**

• **BALISTRERI, Marcello**
**52066 Aachen (DE)**
• **STAPERT, Hendrik**
**52066 Aachen (DE)**

(74) Representative: **Schouten, Marcus Maria**
**Philips**
**Intellectual Property & Standards**
**P.O. Box 220**
**5600 AE Eindhoven (NL)**

(56) References cited:
**EP-A- 0 967 291          WO-A-00/62247**
**WO-A-02/10450          WO-A-92/10587**
**US-A- 5 885 840          US-A1- 2002 172 975**
**US-A1- 2002 185 610          US-A1- 2003 170 914**

**Description**

The invention relates to a method and an apparatus for the detection of occupied binding sites in a sample that contain at least one labeling element.

The US 6 327 031 B1 discloses an optical apparatus derived from a Compact Disc (CD) reading and writing device that is adapted to examine biological, chemical or biochemical samples on a rotating disc. The device detects target specific responses of opaque, reflecting or fluorescent spots of a target substance distributed on said disc that are generated when said spots are irradiated with a beam of laser light. The disc may further carry a code on its surface that allows to (re-)locate specific positions on the disc.

In general, the purpose of a biosensor is to detect the presence and/or concentration of a target substance in an analyte. This detection is based on a specific binding to a "binding site" or capture probe which is immobilized on a substrate. In order to make this binding detectable a label element (or short "label") is attached to the target. The signal of the label needs to be detected with the highest possible sensitivity. There are different approaches to build such an assembly of capture probe - target - label (e.g. one can first attach the label to the target and then let that couple bind to the capture probe or one can first bind the target to the capture probe and in a second step label the immobilized targets). This is relevant if one wants to measure while the binding reaction is still going on, or for the problem of background signal from the solution and the required washing steps to remove non-specifically bound targets and/or labels. Though the presence of labels is measured, one is only interested in the labels which are attached to a target which is immobilized by a capture probe on a substrate.

US2003170914 A1 discloses a method for optically monitoring specific binding ligands immobilized on the surface of a solid support and in which a plurality (e.g., ten) different oligonucleotides can be prepared, each having a different, known sequence complementary to an address sequence on the surface and each having a corresponding distinguishable fluorophore. The labelled probes can be simultaneously applied to the surface and hybridized, e.g., at 55°C for 30 minutes. The surface is then rinsed and scanned to identify the locations of each of the ten sequences. Another set of ten sequences is then hybridized to the surface which is scanned again to identify the second set of ten sequences. This process is continued until all of the locations are identified, after which the master array is stripped by immersing in boiling water for two minutes. In view of the modus operandi of this method it is implicit that maximally one occupied binding site is present within the scanning beam spot at any one time.

Based on this situation it was an object of the present invention to provide means for the detection and quantification of a target substance or a labeling element in a sample with a high sensitivity and reliability.

This object is achieved by a method according to claim 1. Preferred embodiments are disclosed in the dependent claims.

The method according to the present invention allows the detection of occupied binding sites in a sample, wherein the "occupation" of a binding site by definition means that the binding site contains at least one labeling element (for example a certain fluorescent molecule). In the most simple case, a "binding site" may just be a certain location in the sample, the "binding" being the presence of a labeling element at said location. Other examples of (occupied) binding sites will be discussed in connection with preferred embodiments of the invention. The method comprises the following steps:

a) The scanning of said sample with a spot of (preferably electromagnetic) radiation, wherein the effective extension of the spot and the effective scanning speed of the spot are such that nearly always maximally one occupied binding site is present within the currently examined spot area during the corresponding examination duration.

The spot of radiation may for example be the focus of a (laser) light beam produced by an optical pickup unit (OPU) which is scanned above the sample without mechanical contact to the sample. The "extension of the spot" has to be defined reasonably with respect to the shape of the spot, for example as the maximal or the mean diameter of the spot (corresponding to the usual diameter in case of a simply circular spot). Similarly, the "scanning speed" has to be defined reasonably with respect to the particular method of scanning, for example as the average spatial velocity (in m/s) with which the spot moves through the sample. Finally, the "corresponding examination duration" of a spot area in the sample is defined as the (average) time for which points of this area are irradiated by the spot without interruption. If for example a spot of radiation moves stepwise through the sample, the "corresponding examination duration" is the time between two jumps. If the spot moves continuously, the "corresponding examination duration" can be defined as the mean irradiation time of a spot area in the sample (i.e. the average over all its points).

The postulated condition for spot extension and scanning speed means that almost always (i.e. in more than 90%, preferably more than 99% of the time) none or at most one occupied binding site is irradiated by the spot at a time, wherein the consideration of scanning speed takes into account that the appearance of occupied binding sites may be a dynamic process. The proposed scanning may thus be called "digital" because there are effectively only two possible outcomes of a measurement at a location in the sample, i.e. "empty" or "single occupied".

The postulated condition is fulfilled irrespectively of scanning speed in the special case in which the effective extension of the spot is (e.g. by a factor of about $10^{-1}$ to $10^{-5}$) smaller than the mean distance between occupied binding sites. This corresponds to the situation of a sparse distribution of isolated occupied binding sites, in which - apart from seldom exceptions - maximally one occupied binding site is irradiated by the spot at a time.

b) The memorization of each location, which was examined by the aforementioned spot of radiation, as a "candidate" for an occupied binding site if a target specific response was observed from said location. The concrete definition of a "target specific response" depends on the kind of binding sites and labeling elements that are examined. If the labeling element comprises for example metal particles, a target specific response to the radiation of the spot may be the reflection of the scanning light. In case of a fluorescent labeling element, the specific response may be the stimulated emission of fluorescent light, and in case of chemiluminescence, the target specific response may be spontaneously produced light observed in the spot area.

c) The at least one times repeated scanning of locations of the aforementioned candidates. This means that each location of a candidate is measured at least two times such that a temporal development of a potential binding site can be observed. Between repeated scans certain treatments, like washing steps and/or temperature changes may be carried out for instance as stringency test to better discriminate between specific and non-specific binding.

d) The classification of a candidate as a "detected" (or "real") occupied binding site if it shows a predetermined response behavior in the aforementioned repeated scans. A candidate may for example be classified as a detected occupied binding site if it shows the target specific response in all or e.g. in more than 90% of the repeated scans. As will be discussed in more detail in connection with preferred embodiments of the invention, such a classification allows a discrimination between specific and nonspecific binding in a (bio-)chemical sample.

The method described above provides a very sensitive and reliable determination of occupied binding sites within a sample because only one binding site is measured at a time and because the classification of a location as detected occupied binding site is based on repeated measurements. It should be noted that the method optionally comprises the detection of more than one kind of occupied binding site, for example of binding sites containing different labeling elements (e.g. two different fluorophores).

The sample in which the measurements are made may be (approximately) two-dimensional or three-dimensional.

It may preferably comprise a solid surface on which probes are distributed as "binding sites" that are capable to bind directly or indirectly the at least one labeling element. The solid surface may for example be realized by a polymer carrier to which (biological) capture molecules are attached with a surface density of typically between 1 and $10^6$ per $m^2$, preferably between 10 and $10^4$ per $m^2$, wherein said molecules specifically bind a labeling element that shall be detected. An indirect binding of labeling elements may particularly take place via a prior specific binding of a target substance. Said target substance may for example consist of biological molecules of interest in a solution. These biological molecules are then immobilized on the solid surface by capturing them with the probes. To see if the capturing has occurred, a signal from the presence of the targets is needed. This is achieved by attaching a label element (e.g. a fluorescent molecule) to each occupied binding site. The sensitivity of the measurement then depends on the label element (in the case of fluorescence on the number of dye molecules in the label; the more dye molecules, the higher the signal). The biological binding of the target substance to the probes can be due to hybridization of a strand of cDNA, or by recognition of a protein to an antibody, etc. The label element may be bound to the target by a similar biological interaction between specific molecules attached to the real label (e.g. on the surface of a PS sphere containing fluorescent dye molecules). The labeling of a target substance can be carried out in solution before binding to the probes (by mixing in the label elements and incubating) or in a separate step after binding of the target molecules to the probes (by applying a solution containing the labels to the solid surface where target molecules have already bound). The labels may also be included in the target substance, for instance if the target substance is the product of a PCR (multiplication of single DNA strands) in which nucleic acids are supplied with an attached dye molecule. In the latter case, the compound of labels and target substance can formally considered as a "labeling element" in the sense of the present invention.

According to a further development of the aforementioned embodiment, the solid surface of the sample is exposed to a solution that potentially contains the at least one target substance and/or labeling element before or while the sample is scanned with the spot of radiation. The term "target substance" shall comprise in a broad sense any material object one is interested in, for example atoms, ions, molecules, complexes or biological systems like cells or microbial organisms. The target substance and/or labeling element may leave the solution and bind to the probes on the solid surface, thereby being fixed to a certain location for the subsequent measurements. Additional washing and labeling steps (in a sandwich type of assay) can be carried out to improve the specificity of the biological binding.

In the aforementioned method it is particularly possible to determine the concentration of the at least one target substance and/or of the labeling elements in said solution from the measured distribution of detected occupied binding sites on the solid surface of the sample. Thus it is not only possible to detect the mere presence of a target substance and/or labeling element, but also to quantify its amount and spatial distribution. This quantification is based on the effect that the binding of the solved target substance and/or labeling elements to the probes on the solid surface is either a kinetic process or a thermodynamic equilibrium according to which the density of occupied binding sites at a certain time is proportional to the concentration of the target substance and/or labeling elements in the solution. Therefore, the detected density or distribution of occupied binding sites allows inferring the concentration of the target substance and/or labeling elements in the solution. As the proposed method is very sensitive and based on the detection of single occupied binding sites, it is possible to measure extremely low concentrations (typically fM) in this way. The lower detection limit is, among other, determined by the area of the surface covered with capture probes - the larger the surface, the higher the probability to find a single target - at the expense of scanning time. It is an important advantage of this approach, to be able to increase the surface area and thus detection limit without affecting the noise or background.

The labeling elements may in principle be any entity that is capable to bind to a binding site mechanically, electrically, chemically or otherwise. Preferably, the labeling elements comprise a single molecule (particularly a protein or single strand DNA), a collection of a plurality of (identical or different) molecules, preferably a collection of between 10 to $10^8$ molecules, and/or a semi-conducting particle. If the labeling element is a collection of several molecules, a correspondingly stronger response to the irradiation and a better signal-to-noise ratio can be achieved.

The target specific response may in principle be any event or process at the location of the spot that can be detected with appropriate means. In a preferred embodiment, the target specific response comprises the emission of fluorescent light that is stimulated by the radiation of the spot and/or of light generated by chemiluminescence. In this case both the radiation of the scanning spot and the response of light from fluorescence or chemiluminescence can be processed by an optical system without mechanical contact to the sample. Another advantage of the described method is that it does not require the absolute measurement of light intensity from fluorescence or chemiluminescence but only the detection if said intensity is above or below a given threshold, which discriminates the response of occupied binding sites from background. The fluorescence may for example originate from the probes that bind a labeling element, from the labeling element, or from fluorescent markers attached to the labeling element. Moreover, the fluorescence may be the "normal" behavior of a probe that is suppressed or reduced when a labeling element is bound. In this case, the target specific response is the observed reduction in fluorescence. When chemiluminescence is observed, the production of light is chemically induced, and the spot of radiation is only needed to determine the coordinates of the currently examined location.

In the aforementioned embodiment, the sensitivity of the whole method depends on the capability to detect the fluorescent light and to discriminate it from background radiation. In order to produce a strong fluorescence signal without bleaching the fluorescent substance excessively, it is therefore preferred to adjust the parameters of the examination, particularly the intensity of the radiation in the scanning spot such that about 10 % to 90 %, preferably about 30 % to 80 %, of the saturation level of the fluorescence is produced. Said saturation level is defined as the maximum achievable intensity of fluorescence which cannot be increased by a higher intensity of exciting radiation. As the spot of scanning light is typically very small in the proposed method (because single binding sites shall be detected), it is generally no problem to produce the required high intensity that stimulates the desired amount of fluorescence.

According to a further development of the invention, the definition of a "target specific response" is adapted based on the measured responses from scanned locations. In case of the embodiment using fluorescent light, the definition of a target specific fluorescent response typically comprises the setting of a threshold of measured intensity above which a response is classified as "target specific". The optimal value of this threshold depends on the level of background radiation that is present and that has to be discriminated from a proper response of an occupied binding site. It is therefore preferred that the intensity coming from "empty" locations without an occupied binding site is continuously measured and taken as an indication of the level of background radiation.

The proposed method allows to draw conclusions about a sample based on the detection (or absence) of as few as one single binding site. In order to improve the reliability and the statistics of a measurement, however, it is preferred to design a measurement such that about 100 to 1000 occupied binding sites will be detected in a sample during the whole examination duration. A preferred parameter that can be adjusted to achieve these numbers is the size of the sample, e.g. the area of surface covered with capture probes. If for example the concentration of a labeling element in a solution and the density of probes on a solid surface are given, a certain number of occupied binding sites per unit area of the surface and unit time results after a contact between the surface and the solution. In order to achieve the desired numbers of occupied binding sites in a measurement, the (scanned) area of the solid surface has therefore to be chosen appropriately.

The disclosure further relates to an apparatus for the detection of occupied binding sites in a sample, wherein said occupied binding sites contain at least one labeling element, comprising:

- A scanning unit that is adapted to scan the sample with a spot of radiation, wherein the extension and the scanning speed of the spot can be adjusted such that approximately always at most one occupied binding site is present within the currently examined spot area during the corresponding examination duration. The scanning unit may particularly comprise a laser for generating a light beam and focusing it to a circular spot of about 0.1 to 10 $\mu$m diameter.

- A detection unit that is adapted to detect a target specific response from locations in the sample examined by the aforementioned spot. The detection unit may particularly comprise an optical system for collecting (reflected, transmitted, luminescent, ...) light emerging from the sample and a detector to measure its intensity. The detection unit and the scanning unit are preferably integrated in an optical pickup unit (OPU) which is scanned over the sample. Moreover, the units typically include means for focusing and tracking the light beam that produces the spot of radiation (structures on the sample and appropriate signal processing and control, like in a optical pick-up unit, but preferably with not rotating sample).

- An evaluation unit that is adapted to memorize locations from which a target specific response was observed as candidates for an occupied binding site and to classify such a candidate as detected occupied binding site if it shows a predetermined response behavior in repeated scans. The evaluation unit may particularly comprise a data processing unit with the usual components (microprocessor, memory, I/O interfaces etc.) and with appropriate software to execute the required processing steps.

The aforementioned apparatus is able to execute all the steps of a method of the kind described above. Therefore, reference is made to the preceding description for more information on the details, advantages and improvements of that apparatus. Particular embodiments of the apparatus will also be described in more detail below with reference to the Figures.

The apparatus may especially be derived from a Compact Disc player/writer. Moreover, it may comprise a specific carrier for the sample that allows to identify locations in the sample with sufficient spatial resolution and reproducibility. Such a carrier may for example resemble a conventional Compact Disc (CD, including derivates like DVD and the like), i.e. use similar features as found on a DVD and also a similar light path. In contrast to a CD/DVD, the carrier would preferably not be rotated and not be circular (particularly not with a diameter of about 12 cm), but rather have a credit card format with pregrooves (and wobble) for position information and autofocusing of the beam.

For the scanning of a sample with a spot it is possible to move the scanning unit or a part of it with respect to a stationary sample, to move the sample with respect to a stationary scanning unit, to move both sample and scanning unit (for example in transverse directions), to guide a light beam across a sample by moving/rotating polygon mirrors, or the like.

The above and other aspects of the invention will be apparent from and elucidated with reference to the embodiment(s) described hereinafter.

In the following the invention is described by way of example with the help of the accompanying drawings in which:

Fig. 1    schematically shows a perspective view of an apparatus for detecting occupied binding sites in a sample;
Fig. 2    shows an enlarged perspective view of the surface of a sample comprising pre-grooves for tracking a laser beam;
Fig. 3    shows a section across line III-III of Figure 2;
Fig. 4    shows the optics of a confocal measurement.

The embodiment of the invention that will be described now in more detail relates to the challenge of a quantitative and sensitive measurement of the concentration of a biological component (the "target substance") in a liquid mixture. This is usually done by detecting the occurrence of a selective binding of the target to a capture probe, which is attached to a solid surface. The occurrence of the binding is detected by the presence of a label element (or simply "label"), which is attached to the target, or is present on a second (or third) probe that selectively binds to the bound target (or target-probe complex). The gold standards in this type of analysis are fluorescence, which is stimulated by irradiation of the label with light, and chemiluminescence, which is stimulated by a chemical or enzymatic reaction. One of the prime challenges is to have a very low detection limit. This detection limit of a certain test is determined by the affinity and selectivity of the biological interaction (cross-reactivity, non-specific adsorption, binding constant, etc.) as well as by the sensitivity of the sensor or detector (how many events are required to give a significant signal). Typically, the detector is measuring the fluorescent intensity, which is emitted from a surface, while being irradiated by the excitation beam (for instance by the evanescent field of a light guide to which the biomolecular probes are attached). This intensity can be measured by a diode or in case the biomolecules are attached in a patterned fashion (multiplexed) by a CCD camera. For a low concentration of the target molecules in the sample, the density of labels indicating occupied binding sites on the surface is low so that the measured intensity is affected by other sources, like fluorescence of the substrate and all other materials in the light path. The lower detection limit is determined by the accuracy of the background measurement and its subtraction. A significant background signal can be expected from the non-specific binding of labels to the surface or the presence of such labels in close vicinity of the surface. The latter makes it difficult to measure "real time", meaning while the binding reaction is occurring rather than after completion of the reaction step and stringency washing.

Normally, only the integral of the bound labels is detected (as intensity per unit area or integrated optical density, i.e. intensity per unit area multiplied by the area). In this way no differentiation is possible between local variations, specific vs. non-specific binding, and the calibration is difficult as it depends on many parameters, which together determine the background signal. This is overcome by "digital detection" of single events as described below. The invention also provides a very convenient and cost effective detection technology. Implementations are given with a scanning laser beam resembling an optical pick-up unit as it is used for optical data storage systems. Such an arrangement allows the measurement of the number and the coordinates of bound labels (occupied binding sites). In this way the background subtraction is not critical anymore and the lifetime of the binding events allows the distinction between specific and non-specific binding and in this way yields much more accurate and detailed information about the biochemical reaction going on at a conditioned surface. It also allows the measurement of the binding and dissociation constants simultaneously, which is very important for the characterization of biochemical assays.

Existing solutions make use of surface selective illumination of labels to increase selectivity. This is done by evanescent field illumination (e.g. WO 2004/023143 A2, Duveneck et al. Proc. SPIE, vol. 2928 (1996)). The achievable intensities of these methods are far below the saturation limits of the dye molecules. Other existing solutions make use of confocal scanning beams for illumination. In this way the illuminated area is limited and consequently the background radiation and cross-talk is reduced. High spatial resolution and sensitive measurement lead to very long "scanning" times. As an alternative the use of arrays of lightpaths (including source, optical elements and detectors) has been proposed in the US 6 437 345 B1. This, however, is a relatively expensive solution and requires a precise alignment of the cartridge with the reader.

Another method to reduce background fluorescence from the substrate or other components is the use of electrical, chemical or enzymatic triggers to stimulate emission of light. These processes are called electroluminescence or chemiluminescence and do not require stimulation with an irradiating light beam. Instead at least one substrate is added that can cause a reaction that leads to the generation of light.

The fundamentally different approach which is proposed here is based on the detection of single events or "occupied binding sites" and the coordinates of each event rather than detecting the (integral/average) luminescent intensity. The basis for detection of single binding events is a sufficiently strong signal, which can be distinguished unambiguously from the background intensity. By scanning a surface on which binding events are very rare (less than 1 ppm depending on the scanning speed and the spot diameter) one can determine the signal level of the background very accurately because the vast majority of measurements is background. Most of the noise sources do not have a high spatial or timely variation. Only when the signal is above a certain threshold value it is identified as a (potential) binding event and the coordinates of that point are recorded as a candidate of an occupied binding site. In this way a map can be constructed of the potential occupied binding sites and the scanning can be repeated. By continuously comparing the maps, the lifetime of bound sites can be determined and the long-lived can be identified as the specific binding events or occupied binding sites. Short-lived sites can be discarded as noise or unspecific binding. This approach thus provides much more information and more certainty of the measured binding. This is achieved by the adjustment of the size of the scanning spot and the brightness of the illuminated label. Scanning speed and label size are related and can be optimized for each application.

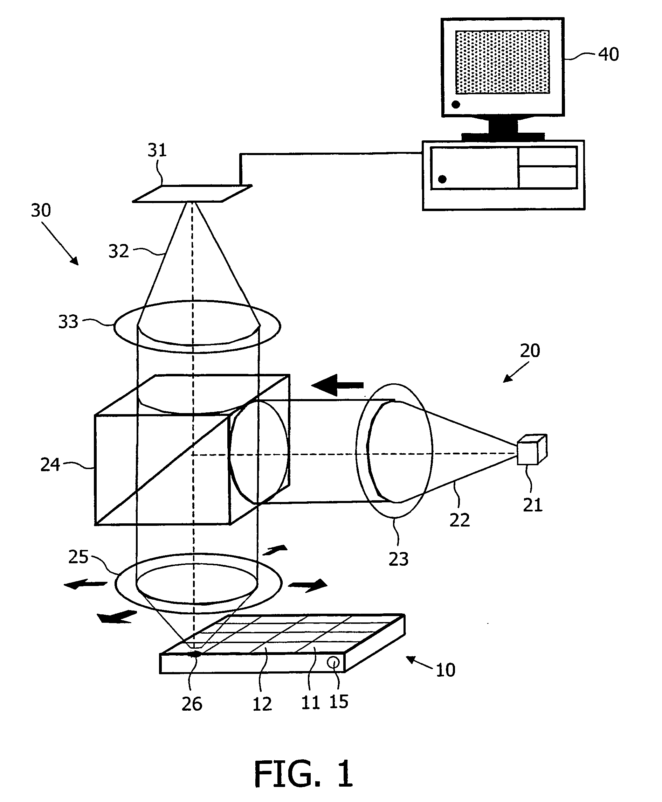

One example of an apparatus using the principles discussed above is based on a DVD optical pickup unit and is schematically shown in Figure 1. It is adapted to detect fluorescence coming from occupied binding sites 15 in a "two-dimensional" sample 10 (of which only a small fraction is shown).

The apparatus comprises a scanning unit 20 with a laser source 21, a first lens 23 (wherein the term "lens" here and in the following also comprises optical systems with several individual lenses) that collimates a laser beam 22 emerging from the laser source 21 to a parallel light bundle, a dichroic beam splitter 24 that reflects the laser beam 22 in a right angle towards the surface of the sample 10, and a second lens 25 (objective) that focuses the laser beam 22 to a spot 26 in the sample 10.

For the detection of fluorescent light coming from the sample 10, the apparatus comprises a detection unit 30 with the following components: the already mentioned lens 25 that collects fluorescent light emerging from the sample 10 and collimates it to a parallel beam which is sent through the beam splitter 24; a third lens 33 that focuses the beam 32 on a detector 31 which is adapted to measure the intensity of incident (fluorescent) light and to produce a corresponding electrical signal.

Finally, the apparatus comprises an evaluation unit 40 that may for example be realized by a conventional computer. The evaluation unit 40 executes all the required processing of the measured data which is described in more detail below. Moreover, the evaluation unit 40 may be adapted to control the apparatus, i.e. to command measurements in certain locations and/or with certain parameters.

A numerical aperture (NA) of the objective 25 of 0.2 at 650 nm excitation may for example be used to obtain a spot 26 with a surface area of $\sim$10 $\mu m^2$ (corresponding to a diameter d of the spot of about 3.6 $\mu m$). If every partition

of the sample with an area of $0.1 \times 0.1$ mm$^2$ is statistically labeled with one label, it may be divided in 1000 virtual sub-partitions 11, 12, ... of $3.3 \times 3.3$ μm$^2$ that can separately be scanned by the aforementioned laser spot 26. For a concentration of 1 pM of target molecules (e.g. proteins) the calculated effective binding rate or "hit rate" is $10^{-4}$ events per μm$^2$ and per second. During a period of 1000 s on average 1 specific binding event will then be measured if one sub-partition per second is scanned.

The sensitivity of the method and the apparatus described above does not depend on the number of events (i.e. detected occupied binding sites) but on the certainty with which a single event can be identified as such. There is no lower limit for the concentration which can be detected except for the reasonable timescale of the duration of the measurement. For a certain optical arrangement it will even be possible to measure during the binding reaction while the unbound labels are still present in the solution above the sensing surface. These aspects will now be explained in more detail.

The basis of the proposed approach is the single event detection in a scanning optical arrangement. Single event detection requires a certain minimum power and energy of the emitted radiation to be detected by a sensor. As a first aspect in this respect, the fluorescent saturation intensity and power shall be considered. The average fluorescence lifetime $\tau_{\text{fluor}}$ of fluorophores is of the order of 2 ns (cf. S.W. Hell and J. Wichmann, Opt. Lett. 19, 780 (1994)). Typical values of the cross sections for the absorption $\sigma_{\text{abs}}$ of these specimens range between $10^{-16}$ and $10^{-17}$ cm$^2$. The saturated fluorescent excitation intensity is

$$I_S = \frac{hc}{\lambda \tau_{\text{fluor}} \sigma_{\text{abs}}} \tag{1}$$

with $h$ the Planck's constant, c the speed of light, and $\lambda$ the wavelength of the absorbed light. A saturated fluorescent excitation intensity $I_S$ of 1.5 MW/cm$^2$ or 15 kW/mm$^2$ is found for $\lambda$ is 650 nm, $\sigma_{\text{abs}}$ is $10^{-16}$ cm$^2$, and $\tau_{\text{fluor}}$ is 2 ns (M.A. Kramer, W.R. Tompkin, R.W. Boyd, Phys. Rev. A 34, 2026 (1986)). Labeled biological specimens should be excited close to, but below the saturation level to obtain optimal fluorescence emission and thus an optimal signal-to-noise ratio (SNR). A save excitation level would be at 60 % to 70% of the saturation level. An optimal excitation power for biological specimens of 2 mW, 100 mW, and 10 kW is found on a 0.2 μm$^2$, 10 μm$^2$ and 1 mm$^2$ surface area, respectively, wherein a surface area of 0.2 μm$^2$ corresponds with an optical spot size of a DVD optical pickup unit (0.6 NA, 650 nm). In a fluorescent multilayer storage technology assessment (cf. WO 01/06501 A2) an excitation power of 2 mW has been used to optimize the fluorescent emission from dye molecules using a DVD optical pickup unit (0.6 NA, 650 nm).

The maximum SNR is obtained and increasing the laser power does not improve the SNR anymore. It should be noted that the maximum number of excitation cycles for an organic fluorophore is on average $10^5$ before it will be bleached. The average total lifetime of an organic fluorophore is

$$T = \tau_{\text{exc}} N = \left( \frac{hc}{\lambda \sigma_{\text{abs}} I} \right) N \tag{2}$$

with N the maximum number of excitation cycles, $\tau_{\text{exc}}$ the lifetime of one excitation cycle at an excitation intensity $I$. The lifetime of one excitation cycle is inversely proportional to the excitation intensity and equals the fluorescent lifetime at saturated intensities ($\tau_{\text{exc}} \approx \tau_{\text{fluor}}$ for $I \approx I_S$). The average total lifetime of an organic fluorophore at saturation levels is 0.2 ms for an average fluorescence lifetime $\tau_{\text{fluor}}$ of fluorophores of the order of 2 ns.

Typical dyes are those from the xanthene and cyanine families, having excitation and emission properties spanning the visible spectrum. These dyes are traded under several names, such as Bodipy, Alexa and Cy-dyes (the applied trade name depends on the exact chemical composition of the dye). Dyes especially suited for laser excitation are known as well. For example, Oregon green 488 and 514 can be excited at 488 and 514 nm, respectively, and are exceptionally photo-stable.

Quantum efficiencies of many commercial dyes are between 0.1 and 1. However, many unsubstituted fluoresceines and rhodamines (e.g. Texas Red) bleach relatively fast (for Texas Red 1% in 10 s at saturation intensity). Substituted dyes, such as tetramethyl rhodamine (TMR) have a higher photo stability, but the fluorescent quantum yield of TMR is lower as compared to the fluorescein conjugates.

Sometimes phosphorescent emission may be preferred (for example to reduce background fluorescent emission). Typical dyes with a relatively late emission are eosins and erythrosines. The quantum yield of these phosphorophores are lower, typically 10 % - 20 % of those of the fluoresceines.

Other suitable dyes contain metal complexes, such as those from Eu, Pt, Cu, Zn, Tb, Dy, Sm, Yb, Nd, Er, Ho, Gd and Ce. Also lanthanide complexes such as based on Ru, Os, Ir, Pd, Re have suitable emission properties.

An increase of fluorescent emission can also be achieved when using fluorescent beads of a certain size. These beads usually consist of a polymer in which fluorophores are dispersed or chemically linked. Typical polymers applied are polystyrene and dextran. These beads show high photo stability. It is also possible to select different dyes such that excitation can be performed at the same wavelength, but emission occurs at a chosen wavelength. By careful selection of the dyes one can use fluorescence resonance energy transfer (FRET) between the dyes to obtain the desired wavelengths. Bead sizes range from 20 nm to several microns. Typically polystyrene beads of 20 nm contain about 180 low molecular weight fluorophores; 200 nm beads about $1.1 \times 10^5$ fluorophores and 1 micron beads about $1.3 \times 10^7$ fluorophores. High signal amplification can in principle be obtained.

Small semi-conducting particles ("Quantum dots and rods") are also suitable labels because they can withstand high laser powers before bleaching occurs. Quantum dot sizes are typically between 1-5 nm (diameter). The emitted wavelength is a function of the particle size (blue to red with increasing diameter), while the absorption spectrum does not change much. Typical materials for Quantum dots are: CdSe, CdTe, etc. Core shell type Quantum dots have also been described, e.g. particles with a CdSe core and ZnS shell. Quantum dots may also be excited electrically or chemically. Quantum rods may be of especial interest as they can emit linear polarized light when their spatial and/or rotational motion is disrupted (e.g. upon binding).

Concerning the size of the laser spot 26, the targeted concentration range of the application is important. For a concentration of 1 pM of target molecules (e.g. proteins) one can expect that the effective binding rate/hit rate will be less than $10^{-4}$ events (occupied binding sites) per $\mu m^2$ and per second. For a reasonable assay accuracy one would like to have 100-1000 events (for reliable statistics and dynamic range). With a sensor area of $100 \times 100$ microns, a measuring rate of one event per second seams to be reasonable. This means that after 100-1000 s the assay would be finished. 100-1000 bound labels need to be detected then on an area of $10^4$ $\mu m^2$, which corresponds with a density of 0.01-0.1 per $\mu m^2$. This means that with a beam area of 1 $\mu m^2$ the limit of statistically single events in the beam is reached. The power of this approach is that it can be easily extended to lower concentrations or shorter times. Due to the single event sensitivity it is possible to measure already 1 fM on the same surface area in the same time (this corresponds to 1 binding event on $10^4$ $\mu m^2$ in 1000s).

For an increased detection limit larger areas can be covered with receptors. In this way the time for 1000 events is reduced proportionally. The scanning time will increase. This can be compensated by an increase in the spot size and power of the spot. Therefore the speed of detection will not be affected and neither will be the sensitivity of the read-out.

Conventional fluorescent-based biosensors illuminate the whole sample surface, e.g. 1 mm$^2$, using propagating wave excitation or evanescent wave excitation. The maximal SNR for propagation wave and evanescent wave excitation is obtained using an excitation power of ~10 kW and ~2 W, respectively. These required powers are not feasible in a commercial biosensor, thus much lower excitation powers are used resulting in a lower potential sensitivity of the biosensor.

Concerning the scanning aspect, numerous optical scanning solutions exist in the optical recording technology. As an example a continuous groove can be present in the substrate surface which is in contact with the mixture to analyze. The groove contains information about the position (a so-called "wobble"). The scanning is achieved either by moving the stage on which the cartridge is mounted in which the (bio-)chemical reaction of a liquid or gaseous mixture and the receptor surface takes place, or alternatively by moving the optical pick-up unit (containing light source, optical elements and detector, as described above). The latter can be achieved by a 2D translation stage or alternatively by an actuated mirror and a stationary light source and detector. The movement can be a linear scanning, like reading a page, or a continuous trace, like in a Compact Disc. Movement of the light beam is preferred over movement of the cartridge as in the latter case acceleration and deceleration will affect the fluid movement inside the sensor.

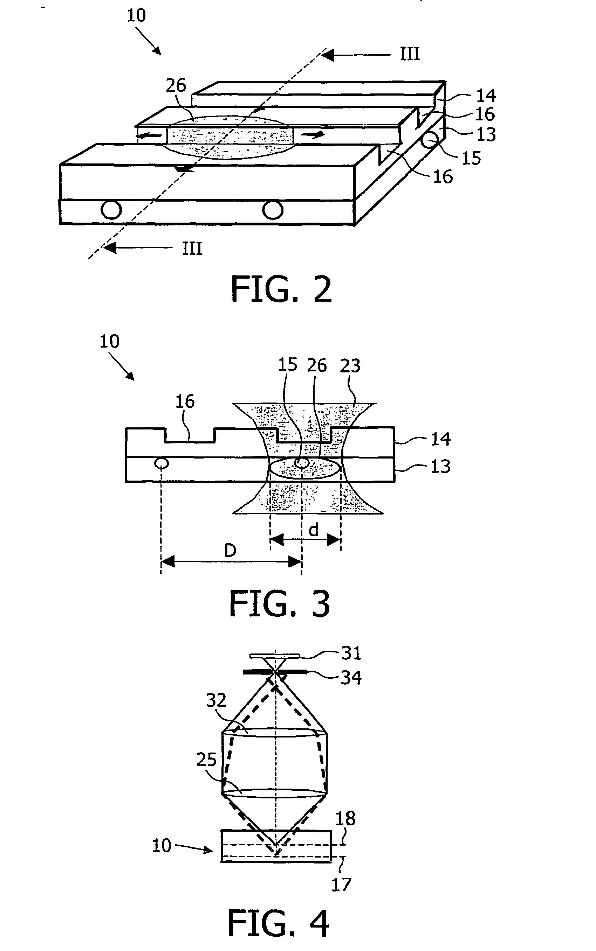

In the example of the proposed scanning fluorescent biosensor based on a DVD optical pickup unit shown in Figure 1, pre-grooves in the sample can be used to guide the spot 26 using the conventional servo-techniques as used in a DVD player for focusing and tracking. Figures 2 and 3 show such pre-grooves 16 for tracking in an enlarged perspective view and a section, respectively. The sample 10 consists of a lower layer 13, which contains the occupied binding sites 15, and an upper layer 14, which comprises the pre-grooves 16. The pre-grooves 16 should be transparent for the excitation and fluorescent light. The depth of the grooves should be tuned to optimize the focus and tracking signals.

For pM concentration during a period of 1000 s on average 1 specific binding event will typically occur within a spot 26 with a size of 10 $\mu m^2$. The scanned spot allows the local detection of one specific binding (occupied binding site). Furthermore, the long lifetime of the specific binding of several days allows fast scanning of the optical spot, along the $10^5$ sub-partitions 11, 12, enabling not only localized measurements, but also time-resolved measurements. For example, the whole sample 10 of Fig. 1 could be scanned 100 times during a total measuring period of 1000 s. This corresponds with a scan speed of 0.3 m/s (the speed of a $1 \times$ DVD is 4.8 m/s). The time-resolution can be used to discriminate between specific binding events, with a lifetime of several days, and non-specific binding events, with much shorter lifetimes in the order of minutes. A higher SNR and sensitivity is obtained due to the localized and time-resolved measurements. The background contribution from sub-partitions 12, ... where no binding event occurred can be subtracted from the measurement. This background reduction due to the localized measurement improves the SNR. The background contribution from non-specific or random events can also be distinguished from the measurement. This background reduction due to the time-resolved measurement further improves the sensitivity of the analysis and provides additional information on the binding kinetics. The maximal SNR is obtained using an excitation power of ~100 mW, which is a feasible power requirement for a commercial biosensor (650 nm DVD recording laser diodes have power between 20-200 mW). The power requirement for the proposed scanning fluorescent biosensor is therefore much smaller compared to the prior art biosensors based on propagation wave and evanescent wave excitation.

During the binding reaction also the fluorescence of the unbound labels in the solution will be measured and the measurement is obtained after binding reaction. However, for a confocal optical arrangement it will even be possible to measure during the binding reaction while the unbound labels are still present in the solution below the sensing surface. Figure 4 shows the optics of a modification of the scanning fluorescent biosensor of Figure 1, said modification being based on a confocal DVD optical pickup unit. A pinhole 34 in front of the detector 31 is used to block the light from the out-of-focus area 17. Only the light from the in-focus plane 18 passes through the pinhole resulting in a smaller focal depth or optical depth resolution. Even with a small penetration of the detected volume labels in the liquid will move fast (due to Brownian motion, diffusion or convection). In a repeated scanning their co-ordinates will be different. By correlating different scans they can be eliminated as unbound.

Further modifications of the example of a scanning fluorescent biosensor described above are for example:

- While above a NA of 0.2 was used for the excitation and collection of the fluorescent light, it is also possible to use a much larger NA to collect the fluorescent light, e.g. 0.85 NA, and a lower NA, e.g. 0.2 NA, for the excitation using the same lens. An improvement of the fluorescent collection efficiency with a factor $\left(\dfrac{0.9}{0.2}\right)^2 = 20$ results in a further improvement of the SNR and thus the sensitivity of the biosensor. More details on this approach may be found in the WO 2004 023459 A2.
- In the described example the whole OPU is scanned above the fixed sample. A scanning fluorescent biosensor without translating components can be obtained by using a non-translated tilting mirror, e.g. galvano mirror, while the OPU and the sample are fixed.
- Pulsed operation of the laser can be used to decrease bleaching effects and to measure at the same frequency, thereby filtering processes that occur at other frequencies.
- Measurement in reflection can be done by illuminating through the sample and applying a reflector (e.g. Au) on the receptor surface. The Au reflector can be used directly for the quenching of the fluorescence (molecular beacons). Otherwise any other inactive metal or dielectric surface can be used.
- To increase the dynamic range of a sensor, different spot sizes can be used on the same chip.
- Smart fluorescent probes can be used with an on-off triggering of fluorescence upon binding (e.g. molecular beacons).
- Probes can be used that show fluorescence alone or if a certain label is bound, and that stop or reduce fluorescence after binding of a target substance. In this case, the target specific response to be detected is a "hole" of fluorescence in a generally fluorescent sample.

The scanning fluorescent biosensor proposed here has the following advantages:

- SNR improves compared to prior-art biosensors, due to the optimal fluorescent excitation of the biological specimens close to the saturation levels, due to the localized measurements of the specific binding events, and due to the time- (and place) resolved measurements of the specific and non-specific binding events.
- The method allows detection of binding kinetics.
- The method allows for a "simple" and cheap read-out system (DVD instead of expensive confocal scanners).

Important applications of the described apparatus and method may be in the areas of molecular diagnostics (clinical diagnostics, point-of-care diagnostics), biosensors, DNA and protein arrays (e.g. detection of proteins or gene sequences for molecular diagnostics or screening), cell analysis, drug screening, environmental sensors, food quality sensors, etc., especially where a very high sensitivity and throughput are required.

Finally it is pointed out that in the present application the term "comprising" does not exclude other elements or steps, that "a" or "an" does not exclude a plurality, and that a single processor or other unit may fulfill the functions of several means. Moreover, reference signs in the claims shall not be construed as limiting their scope.

**Claims**

1. A method for the detection of occupied binding sites (15) in a sample (10), which sites contain at least one labeling element, the method comprising the steps of:

a) scanning the sample (10) with a spot (26) of radiation, wherein the extension (d) and the scanning speed of the spot (26) are such that approximately always maximally one occupied binding site (15) is present within the currently examined spot area during the corresponding examination duration;
b) memorizing each location (11) examined by the spot (26) of radiation as a candidate for an occupied binding site (15) if a target specific response is observed from that location;
c) scanning the locations (11) of said candidates at least one more times;
d) classifying a candidate as detected occupied binding site (15) if it shows a predetermined response behavior in the repeated scanning.

2. The method according to claim 1, **characterized in that** the sample (10) comprises a solid surface with distributed probes on it that are able to bind labeling elements directly or indirectly, preferably via a prior binding of a target substance.

3. The method according to claim 2, **characterized in that** said surface of the sample (10) is exposed before or during the scanning procedure to a solution containing labeling elements and/or target substance.

4. The method according to claim 3, **characterized in that** the concentration of the labeling elements and/or target substance in said solution is determined from the distribution of detected occupied binding sites (15) on said surface of the sample (10).

5. The method according to claim 1, **characterized in that** at least one of the labeling elements is a single molecule, a collection of a plurality of molecules, preferably a plurality of between $10^1$ and $10^8$ molecules, and/or a semi-conducting particle.

6. The method according to claim 1, **characterized in that** the target specific response comprises the emission of light due to fluorescence or chemiluminescence.

7. The method according to claim 6, **characterized in that** the intensity of the radiation of the scanning spot (26) corresponds to about 10 % to 90 % of the saturation level of the fluorescence label, preferably to about 30 % to 80 %.

8. The method according to claim 1, **characterized in that** the definition of the target specific response is adapted based on the measured responses of scanned locations (11, 12).

9. The method according to claim 1, **characterized in that** the size of the scanned sample (10) and the whole examination duration are chosen such that about 100 to 1000 occupied binding sites (15) are detected.


**Patentansprüche**

1. Verfahren zur Detektion von besetzten Bindungsstellen (15) in einer Probe (10), wobei die Bindungsstellen mindestens ein Markierungselement enthalten, wobei das Verfahren die folgenden Schritte umfasst:

a) Abtasten der Probe (10) mit einem Strahlungsfleck (26), wobei die Ausdehnung (d) und die Abtastgeschwindigkeit des Flecks (26) derart beschaffen sind, dass während der entsprechenden Untersuchungsdauer nahezu immer maximal eine besetzte Bindungsstelle (15) innerhalb des aktuell untersuchten Fleckbereichs vorhanden ist;
b) Merken jedes durch den Strahlungsfleck (26) untersuchten Ortes (11) als Kandidat für eine besetzte Bindungsstelle (15), wenn von diesem Ort eine targetspezifische Reaktion beobachtet wird;
c) Abtasten der Orte (11) der genannten Kandidaten mindestens ein weiteres Mal;
d) Klassifizieren eines Kandidaten als detektierte besetzte Bindungsstelle (15), wenn er bei der wiederholten Abtastung ein vorgegebenes Reaktionsverhalten zeigt.

2. Verfahren nach Anspruch 1, **dadurch gekennzeichnet, dass** die Probe (10) eine solide Oberfläche mit darauf verteilten Sonden umfasst, die in der Lage sind, Markierungselemente direkt oder indirekt zu binden, vorzugsweise über ein vorheriges Binden einer Targetsubstanz.

3. Verfahren nach Anspruch 2, **dadurch gekennzeichnet, dass** die genannte Oberfläche der Probe (10) vor oder während der Abtastprozedur einer Lösung ausgesetzt wird, die Markierungselemente und/oder Targetsubstanz enthält.

4. Verfahren nach Anspruch 3, **dadurch gekennzeichnet, dass** die Konzentration der Markierungselemente und/oder Targetsubstanz in der genannten Lösung anhand der Verteilung der detektierten besetzten Bindungsstellen (15) auf der genannten Oberfläche der Probe (10) ermittelt wird.

5. Verfahren nach Anspruch 1, **dadurch gekennzeichnet, dass** mindestens eines der Markierungselemente ein einzelnes Molekül, eine Ansammlung einer Vielzahl von Molekülen, vorzugsweise einer Vielzahl von zwischen $10^1$ und $10^8$ Molekülen, und/oder ein halb-leitender Partikel ist.

6. Verfahren nach Anspruch 1, **dadurch gekennzeichnet, dass** die targetspezifische Reaktion die Emission von Licht aufgrund von Fluoreszenz oder Chemilumineszenz umfasst.

7. Verfahren nach Anspruch 6, **dadurch gekennzeichnet, dass** die Strahlungsintensität des Abtastflecks (26) etwa 10 % bis 90 % des Sättigungsniveaus der Fluoreszenzmarkierung, vorzugsweise ca. 30 % bis 80 %, entspricht.

8. Verfahren nach Anspruch 1, **dadurch gekennzeichnet, dass** die Definition der target-spezifischen Reaktion basierend auf den gemessenen Reaktionen von abgetasteten Orten (11, 12) angepasst wird.

9. Verfahren nach Anspruch 1, **dadurch gekennzeichnet, dass** die Größe der abgetasteten Probe (10) und die gesamte Untersuchungsdauer so gewählt werden, dass ca. 100 bis 1000 besetzte Bindungsstellen (15) detektiert werden.

**Revendications**

1. Procédé de détection des sites de liaison occupés (15) dans un échantillon (10), lesdits sites contenant au moins un élément de marquage, le procédé comprenant les étapes consistant à :

   a) scanner l'échantillon (10) avec un faisceau ponctuel (26) de rayonnement, l'étendue (d) et la vitesse de balayage du faisceau ponctuel (26) étant telles que, quasiment toujours, au maximum un site de liaison occupé (15) est présent dans la zone ponctuelle en cours d'examen pendant la durée d'examen correspondante ;
   b) mémoriser chaque emplacement (11) désigné comme site de liaison occupé (15) candidat par le faisceau ponctuel (26) de rayonnement, si une réponse spécifique de la cible est observée en provenance de cet emplacement ;
   c) scanner les emplacements (11) desdits candidats au moins une fois de plus ;
   d) classer un candidat comme site de liaison occupé (15) détecté s'il présente, lors des balayages répétés, un comportement en réponse prédéterminé.

2. Procédé selon la revendication 1, **caractérisé en ce que** l'échantillon (10) comprend une surface solide sur laquelle sont réparties des sondes qui sont capables de se lier, directement ou indirectement, à des éléments de marquage, de préférence par le biais d'une liaison préalable à la substance cible.

3. Procédé selon la revendication 2, **caractérisé en ce que** ladite surface de l'échantillon (10) est exposée, avant ou pendant la procédure de balayage, à une solution contenant des éléments de marquage et/ou la substance cible.

4. Procédé selon la revendication 3, **caractérisé en ce que** la concentration des éléments de marquage et/ou de la substance cible dans ladite solution est déterminée à partir de la distribution des sites de liaison occupés (15) détectés sur ladite surface de l'échantillon (10).

5. Procédé selon la revendication 1, **caractérisé en ce qu'**au moins un des éléments de marquage est une molécule unique, un ensemble d'une pluralité de molécules, de préférence une pluralité d'entre $10^1$ et $10^8$ molécules, et/ou une particule semiconductrice.

**6.** Procédé selon la revendication 1, **caractérisé en ce que** la réponse spécifique de la cible comprend l'émission de lumière due à la fluorescence ou à la chimio luminescence.

**7.** Procédé selon la revendication 6, **caractérisé en ce que** l'intensité du rayonnement du faisceau ponctuel de balayage (26) correspond à environ 10 % à 90 % du niveau de saturation du marqueur fluorescent, de préférence à environ 30 % à 80 %.

**8.** Procédé selon la revendication 1, **caractérisé en ce que** la définition de la réponse spécifique de la cible est adaptée en se basant sur les réponses mesurées aux emplacements scannés (11, 12).

**9.** Procédé selon la revendication 1, **caractérisé en ce que** la taille de l'échantillon (10) scanné et la durée totale de l'examen sont choisies de sorte qu'environ 100 à 1 000 sites de liaison occupés (15) soient détectés.

FIG. 1

FIG. 2

FIG. 3

FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6327031 B1
- US 2003170914 A1
- WO 2004023143 A2
- US 6437345 B1
- WO 0106501 A2
- WO 2004023459 A2

### Non-patent literature cited in the description

- **Duveneck et al.** *Proc. SPIE,* 1996, vol. 2928
- **S.W. Hell ; J. Wichmann.** *Opt. Lett.,* 1994, vol. 19, 780
- **M.A. Kramer ; W.R. Tompkin ; R.W. Boyd.** *Phys. Rev.,* 1986, vol. A 34, 2026